# EUROPEAN PATENT APPLICATION

(11) **EP 4 516 908 A1**
(43) Date of publication of application: **05.03.2025**
(21) Application number: 23795991.1
(22) Date of filing: 28.03.2023
(51) Int. Cl.: C12N 15/54, C12N 1/15, C12N 1/19, C12N 1/21, C12N 5/10, C12N 9/10, C12P 19/30

(54) **PROTEIN HAVING NAMPT ACTIVITY, AND METHOD FOR PRODUCING NMN**

(30) Priority: 28.04.2022 JP 2022075285
(71) Applicant: Kyowa Hakko Bio Co., Ltd., Tokyo 100-8185 (JP)
(72) Inventor: HORI Kazumasa, Tokyo 100-8185 (JP); UJIHARA Tetsuro, Tokyo 100-8185 (JP); ATAKA Yusuke, Tokyo 100-8185 (JP)
(74) Representative: Vossius & Partner Patentanwälte Rechtsanwälte mbB
(86) International application number: PCT/JP2023/012607
(87) International publication number: WO 2023/210244

(57) **Abstract**

A protein of the present invention is a protein having nicotinamide phosphoribosyl transferase activity, described in any one of the following [1] to [3]:
[1] a protein consisting of an amino acid sequence set forth in SEQ ID NO: 3 or 5;
[2] a mutant protein consisting of an amino acid sequence obtained by deleting, substituting, inserting, or adding 1 to 20 amino acids in the amino acid sequence set forth in SEQ ID NO: 3 or 5; and
[3] a homologous protein consisting of an amino acid sequence having 90% or more identity to the amino acid sequence set forth in SEQ ID NO: 3 or 5.

## Description

### Technical Field

The present invention relates to a protein having nicotinamide phosphoribosyl transferase (NAMPT) activity, and a method for producing nicotinamide mononucleotide (NMN) using the protein.

### Background Art

Nicotinamide mononucleotide (NMN) is a precursor of nicotinamide adenine dinucleotide (NAD) used as an electron carrier in a mammal, is known to have a large number of functions such as activation of mitochondria, and activation of a sirtuin gene, and is expected as a supplement (Non Patent Literatures 1 and 2).

As a method for producing NMN, a chemical synthesis method (Patent Literature 1), a method for enzymatically degrading NAD (Non Patent Literature 3), an extraction method from yeast (Patent Literature 2), and the like are known. These production methods have, however, problems of low productivity and high production cost, and a more inexpensive and efficient production method has been demanded.

As a more efficient production method, a method for producing NMN by condensing, in a bacterial cell, nicotinamide (NAM) and 5-phospho-α-D-ribose 1-phosphate (PRPP) produced by an enzymatic or biological method is known (Non Patent Literatures 4, 5, and 6, and Patent Literature 3). In this condensation reaction between nicotinamide and PRPP, a nicotinamide phosphoribosyl transferase (NAMPT) is used. The NAMPT is known to be involved in the synthesis of NMN also in a human living body (Non Patent Literature 7), and is regarded as a key enzyme of NMN synthesis.

An example of the NAMPT includes a *Haemophilus ducreyi-*derived NAMPT disclosed in Non Patent Literature 4. Besides, Non Patent Literature 5 discloses Nampts derived from *Shewanella oneidensis, Sphingopyxis* sp. C-1, *Chitinophaga pinensis, Homo sapiens, Sus scrofa, Mus musculus, Boleophthalmus pectinirostris, Rhinopithecus roxellana, Pteropus alecto,* and *Xanthomonas translucens,* and discloses that high NMN productivity is exhibited when a Nampt derived from *Sphingopyxis* sp. C-1 or *Chitinophaga pinensis* is used.

### Citation List

### Patent Literature

Patent Literature 1: International Publication No. WO2016/160524
Patent Literature 2: International Publication No. WO2017/022768
Patent Literature 3: International Publication No. WO2019/065876

### Non Patent Literature

Non Patent Literature 1: Kim Baumann, "Counteracting toxic protein aggregation", Nature Reviews Molecular Cell Biology 17 (2016) 679-690
Non Patent Literature 2: Rui-Xiong Huang et al., "Nicotinamide mononucleotide attenuates glucocorticoid-induced osteogenic inhibition by regulating the SIRT1/PGC-1α signaling pathway", Molecular Medicine Reports 22 (2020) 145-154
Non Patent Literature 3: E. Harth et al., "Control of photosynthetic oxygen evolution by the internal Ph of the chloroplast thylakoid Inhibition of photosynthetic oxygen evolution by uncouplers at high Ph and restoration of electron flow by an artificial electron donor for photosystem II", FEBS Letters 42 (1974) 161-164
Non Patent Literature 4: G. C. Marinescu, "β-nicotinamide mononucleotide (NMN)production in Escherichia coli", Scientific Reports 8(2018) 12278
Non Patent Literature 5: Shinichiro Shoji et al., "Metabolic design for selective production of nicotinamide mononucleotide from glucose and nicotinamide", Metabolic Engineering 65 (2021)167-177
Non Patent Literature 6: Kazane Sugiyama et al., "Nicotinamide mononucleotide production by fructophilic lactic acid bacteria", Scientific Reports 11 (2021) 7662
Non Patent Literature 7: Antje Garten et al. "Nampt: linking NAD biology, metabolism and cancer", Trends in Endocrinology & Metabolism 20 (2009) 130-138

### Summary of Invention

### Technical Problem

As described above, although a large number of methods for producing NMN from nicotinamide by expressing a NAMPT derived from a different species in a microorganism such as *E. coli* are known, the productivity is not sufficient. In order to more efficiently produce NMN, it is demanded to search for a NAMPT having high activity and capable of highly efficiently producing NMN.

An object of the present invention is to provide a protein having improved nicotinamide phosphoribosyl transferase (NAMPT) activity, and a method for producing nicotinamide mononucleotide using the protein.

### Solution to Problem

The present inventors have made earnest studies to provide a novel protein having NAMPT activity, and have found that NAMPTs derived from *Bisgaardia hudsonensis* and *Chitinophaga rupis* have hither NMN production activity than conventionally known NAMPTs derived from *Haemophilus ducreyi* and *Shewanella oneidensis,* resulting in accomplishing the present invention.

The present invention relates to the following 1 to 8:
1. A protein, having nicotinamide phosphoribosyl transferase activity, described in any one of the following [1] to [3]:
   [1] a protein consisting of an amino acid sequence set forth in SEQ ID NO: 3 or 5;
   [2] a mutant protein consisting of an amino acid sequence obtained by deleting, substituting, inserting, or adding 1 to 20 amino acids in the amino acid sequence set forth in SEQ ID NO: 3 or 5; and
   [3] a homologous protein consisting of an amino acid sequence having 90% or more identity to the amino acid sequence set forth in SEQ ID NO: 3 or 5.
2. A DNA encoding the protein according to the above 1.
3. The DNA according to the above 2 that is a DNA described in any one of the following [4] to [6]:
   [4] a DNA consisting of a nucleotide sequence set forth in SEQ ID NO: 2 or 4;
   [5] a DNA that hybridizes, under stringent conditions, a DNA consisting of a nucleotide sequence complementary to the nucleotide sequence set forth in SEQ ID NO: 2 or 4; and
   [6] a DNA consisting of a nucleotide sequence having 90% or more identity to the nucleotide sequence set forth in SEQ ID NO: 2 or 4.
4. A recombinant DNA comprising the DNA according to the above 2 or 3.
5. A transformant obtained by transforming a host cell with the recombinant DNA according to the above 4.
6. The transformant according to the above 5 that is a transformant having increased productivity of nicotinamide mononucleotide (NMN) as compared with the host cell.
7. The transformant according to the above 5, wherein the host cell is *E. coli.*
8. A method for producing NMN, comprising producing nicotinamide mononucleotide (NMN) by using the transformant according to any one of the above 5 to 7.
9. A method for producing NMN, comprising producing nicotinamide mononucleotide (NMN) by using the protein according to the above 1.

### Advantageous Effects of Invention

According to the present invention, a protein having improved nicotinamide phosphoribosyl transferase activity can be provided, and an efficient method for producing nicotinamide mononucleotide using the protein can be provided.

### Description of Embodiments

### 1. Protein of the present invention

A protein of the present invention is a protein, having nicotinamide phosphoribosyl transferase activity, described in any one of the following [1] to [3]:
[1] a protein consisting of an amino acid sequence set forth in SEQ ID NO: 3 or 5;
[2] a mutant protein consisting of an amino acid sequence obtained by deleting, substituting, inserting, or adding 1 to 20 amino acids in the amino acid sequence set forth in SEQ ID NO: 3 or 5; and
[3] a homologous protein consisting of an amino acid sequence having 90% or more identity to the amino acid sequence set forth in SEQ ID NO: 3 or 5.

Here, the protein consisting of the amino acid sequence set forth in SEQ ID NO: 3 is a protein having nicotinamide phosphoribosyl transferase activity derived from *Bisgaardia hudsonensis,* and the protein consisting of the amino acid sequence set forth in SEQ ID NO: 5 is a protein having nicotinamide phosphoribosyl transferase activity derived from *Chitinophaga rupis.*

The nicotinamide phosphoribosyl transferase (hereinafter referred to as NAMPT) is an enzyme for condensing nicotinamide (NAM) with 5-phospho-α-D-ribose 1-diphosphate (hereinafter referred to as PRPP) to produce β-nicotinamide mononucleotide (compound name: [(2R,3S,4R,5R)-5-(3-carbamoylpyridin-1-ium-1-yl)-3,4-dihydroxyoxolan-2-yI]methylhydrogen phosphate; β-NMN).

The nicotinamide phosphoribosyl transferase (NAMPT) activity refers to activity of synthesizing β-NMN from NAM and PRPP, or activity of synthesizing β-NMN by using, as a substrate, NAM, PRPP, ATP, and a water molecule.

A mutant protein refers to a protein obtained by artificially deleting or substituting an amino acid residue in an original protein, or artificially inserting or adding an amino acid residue in the protein. In the mutant protein described above in the item [2], for deleting, substituting, inserting or adding the amino acids, 1 to 20 amino acids may be deleted, substituted, inserted, or added in optional positions in the amino acid sequence set forth in SEQ ID NO: 3 or 5, and for example, 1 to 15, 1 to 10, 1 to 5, 1 to 4, 1 to 3, 1 to 2, or 1 amino acid may be deleted, substituted, inserted, or added.

The amino acids to be substituted, inserted, or added may be naturally occurring or non-naturally occurring amino acids. Examples of the naturally occurring amino acids include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Examples of mutually substitutable amino acids are as follows. Amino acids belonging to the same group are mutually substitutable.
Group A: leucine, isoleucine, norleucine, valine, norvaline, alanine, 2-aminobutanoic acid, methionine, o-methylserine, t-butylglycine, t-butylalanine, and cyclohexylalanine
Group B: aspartic acid, glutamic acid, isoaspartic acid, isoglutamic acid, 2-aminoadipic acid, and 2-aminosuberic acid
Group C: asparagine, and glutamine
Group D: lysine, arginine, ornithine, 2,4-diaminobutanoic acid, and 2,3-diaminopropionic acid
Group E: proline, 3-hydroxyproline, and 4-hydroxyproline
Group F: serine, threonine, and homoserine
Group G: phenylalanine, and tyrosine

Homologous proteins refer to a group of proteins that every living thing in nature has, and are derived from proteins having the same evolutionary origin. The homologous proteins are similar to one another in the structure and the function. The amino acid sequence of the homologous protein described above in the item [3] has 90% or more, preferably 91% or more, 92% or more, 93% or more, 94% or more, or 95% or more, more preferably 96% or more, 97% or more, or 98% or more, and further preferably 99% or more identity to the amino acid sequence set forth in SEQ ID NO: 3 or 5.

Identity between amino acid sequences or nucleotide sequences can be determined by using algorithm BLAST by Karlin and Altschul [Pro. Nat. Acad. Sci. USA, 90, 5873 (1993)] or FASTA [Methods Enzymol., 183, 63 (1990)]. Based on the algorithm BLAST, programs designated as BLASTN or BLASTX have been developed [J. Mol. Biol., 215, 403 (1990)]. When a nucleotide sequence is to be analyzed by using BLASTN based on BLAST, parameters are set, for example, as score = 100, and wordlength = 12. Alternatively, when an amino acid sequence is to be analyzed by using BLASTX based on BLAST, parameters are set, for example, as score = 50, and wordlength = 3. When BLAST and Gapped BLAST program are used, default parameters of the respective programs are used. Specific methods for performing these analysis methods are known.

It can be confirmed, for example, by the following method, that the mutant protein of [2] or the homologous protein of [3] has NAPRT activity. First, a recombinant DNA having a DNA encoding the protein to be confirmed for the activity is produced by a method described below. Next, the recombinant DNA is used for culturing a microorganism having no NAMRT activity or β-NMN production activity, for example, a microorganism obtained by transforming *Escherichia coli* W3110 in which a DNA encoding nicotinamidase (pncA gene), a DNA encoding nicotinamide mononucleotide amidase (pncC gene), a DNA encoding acid phosphatase (aphA gene), a DNA encoding 5'-nucleotidase/UDP-sugar hydrolase (ushA gene), or a DNA encoding nicotinamide riboside phosphorylase (deoD gene) is deleted, and nicotinamide is added to the medium to produce β-NMN. Finally, HPLC described below is used to detect β-NMN in a culture supernatant. NAMPT activity can be confirmed by detecting β-NMN.

### 2. DNA of the present invention

A DNA of the present invention is a DNA encoding the protein of the present invention, namely, the protein described above in the item [1], the mutant protein described above in the item [2], or the homologous protein described above in the item [3].

Specifically, the DNA of the present invention can be a DNA described in any one of the following [4] to [6]:
[4] a DNA consisting of a nucleotide sequence set forth in SEQ ID NO: 2 or 4;
[5] a DNA that hybridizes, under stringent conditions, a DNA consisting of a nucleotide sequence complementary to the nucleotide sequence set forth in SEQ ID NO: 2 or 4; and
[6] a DNA consisting of a nucleotide sequence having 90% or more identity to the nucleotide sequence set forth in SEQ ID NO: 2 or 4.

Here, the DNA set forth in SEQ ID NO: 2 is a DNA obtained by codon optimization, for expression in *E. coli,* of a nucleotide sequence (SEQ ID NO: 1) of a gene encoding *Bisgaardia hudsonensis-derived* nicotinamide phosphoribosyl transferase set forth in SEQ ID NO: 3, and the DNA set forth in SEQ ID NO: 4 is a gene encoding *Chitinophaga rupis-derived* nicotinamide phosphoribosyl transferase set forth in SEQ ID NO: 5.

An example of the DNA encoding the protein consisting of the amino acid sequence set forth in SEQ ID NO: 3 includes a DNA having a nucleotide sequence set forth in SEQ ID NO: 2 or 1, and an example of the DNA encoding the protein consisting of the amino acid sequence set forth in SEQ ID NO: 5 includes a DNA having a nucleotide sequence set forth in SEQ ID NO: 4. The DNA encoding the mutant protein described above in the item [2] or the homologous protein described above in the item [3] of the item 1 can be, for example, the DNA described above in the item [4] or [5].

Regarding the DNA described above in the item [5], to "hybridize" refers to a step of hybridizing the DNA with a DNA having a specific nucleotide sequence or a part of this DNA. Accordingly, a nucleotide sequence of the DNA that hybridizes the DNA having a specific nucleotide sequence or a part of the DNA may be a DNA that is useful as a probe for Northern or Southern blot analysis, or has a length usable as an oligonucleotide primer in PCR analysis.

An example of a DNA used as a probe includes a DNA having at least 100 bases or more, preferably 200 bases or more, and more preferably 500 bases or more in length, and an example of a DNA used as a primer includes a DNA having at least 10 bases or more, and preferably 15 bases or more in length.

A method of DNA hybridization experiment is well known, and the experiment can be performed with hybridization conditions determined in accordance with, for example, Molecular Cloning, 4th edition (Cold Spring Harbor Laboratory Press (2012)), Methods for General and Molecular Bacteriology (ASM Press (1994)), Immunology methods manual (Academic Press (1997)), and other many standard textbooks.

Alternatively, also in accordance with a manual attached to a commercially available hybridization kit, a DNA that hybridizes under stringent conditions can be obtained. An example of the commercially available hybridization kit includes Random Primed DNA Labeling Kit (manufactured by Roche Diagnostic) with which a probe is prepared by a random prime method to perform hybridization under stringent conditions.

The stringent conditions can be, for example, the following conditions: A filter having a DNA immobilized thereon and a probe DNA are incubated at 42°C overnight in a solution containing 50% formamide, 5 x SSC (750 mM sodium chloride and 75 mM sodium citrate), 50 mM sodium phosphate (pH 7.6), 5 x Denhardt's solution, 10% dextran sulfate, and 20 µg/L modified salmon sperm DNA, and the resultant filter is washed in, for example, 0.2 x SSC solution at about 65°C.

The above-described various conditions can be set also by adding or changing a blocking reagent used for suppressing the background of the hybridization experiment. The addition of the blocking reagent may include change of the hybridization conditions for conforming the conditions.

An example of the DNA capable of hybridizing under stringent conditions described above includes a DNA consisting of a nucleotide sequence having 90% or more, preferably 91% or more, 92% or more, 93% or more, 94% or more, or 95% or more, more preferably 96% or more, 97% or more, or 98% or more, and further preferably 99% or more identity, which is calculated using the above-described program such as BLAST or FASTA based on the above-descried parameters, to the nucleotide sequence set forth in SEQ ID NO: 2 or 4.

The DNA of the present invention can be obtained, for example, by PCR [PCR Protocols, Academic Press (1990)] in which a probe designable based on a nucleotide sequence of a DNA encoding a protein consisting of the amino acid sequence set forth in SEQ ID NO: 3 or 5 is used; Southern hybridization in a chromosomal DNA library of a microorganism, preferably one belonging to the genus *Bisgaardia* or the genus *Chitinophaga,* and more preferably *Bisgaardia hudsonensis* or *Chitinophaga rupis* is employed; or a primer DNA designable based on a DNA encoding the protein consisting of the amino acid sequence set forth in SEQ ID NO: 3 or 5 is used; and the chromosomal DNA library is used as a template.

The DNA of the present invention can be obtained, for example, by mutating, with a DNA encoding the protein consisting of the amino acid sequence set forth in SEQ ID NO: 3 or 5 (for example, a DNA consisting of the nucleotide sequence set forth in SEQ ID NO: 2 or 4), a nucleotide sequence of a portion, on the DNA, encoding consecutive or non-consecutive 1 to 20 amino acid residues by a site directed mutagenesis method described in, for example, Molecular Cloning, 4th edition (Cold Spring Harbor Laboratory Press (2012)), Current Protocols in Molecular Biology (JOHN WILEY & SONS, INC.) or the like to substitute the nucleotide sequence with a nucleotide sequence encoding another amino acid residue. Alternatively, the DNA of the present invention can be obtained by using Prime STAR Mutagenesis Basal Kit (manufactured by Takara Bio Inc.) or the like.

The DNA encoding the mutant protein, described above in item [2] in 1, consisting of an amino acid sequence obtained by deleting, substituting, inserting, and/or adding 1 to 20 amino acids in the amino acid sequence set forth in SEQ ID NO: 3 or 5, can be obtained, for example, by performing error-prone PCR or the like using the DNA consisting of the nucleotide sequence set forth in SEQ ID NO: 2 or 4 as a template.

Alternatively, the DNA encoding the mutant protein, described above in item [2] in 1, consisting of an amino acid sequence obtained by deleting, substituting, inserting, and/or adding 1 to 20 amino acids in the amino acid sequence set forth in SEQ ID NO: 3 or 5, can be obtained by site directed mutagenesis [Gene, 77, 51 (1989)] by PCR using a pair of PCR primers respectively having, at the 5' terminals, a nucleotide sequence designed to introduce target mutation (deletion, substitution, insertion, or addition).

Alternatively, the DNA of the present invention can be obtained in accordance with a manual attached to a commercially available site directed mutagenesis kit. An example of the commercially available site directed mutagenesis kit includes Prime STAR(R) Mutagenesis Basal Kit (manufactured by Takara Bio Inc.) capable of introducing mutation (deletion, substitution, insertion, or addition) in a position into which target mutation is desired to introduce.

Specifically, first, a pair of mutation introducing primers having 15 bases overlapped on the 5' side is designed by using, as a template, a plasmid including a nucleotide sequence designed to introduce target mutation (deletion, substitution, insertion, or addition). At this point, the overlap portion includes the target mutation. Next, the mutation introducing primers are used to perform PCR by using, as a template, a plasmid having a nucleotide sequence to which the target mutation is desired to be introduced. When the thus obtained amplified fragment is transformed into *E.coli,* a plasmid having a nucleotide sequence in which the target mutation has been introduced can be obtained.

A DNA encoding the homologous protein, described above in item [3] in 1, consisting of an amino acid sequence having 90% or more identity to the amino acid sequence set forth in SEQ ID NO: 3 or 5, can be obtained, for example, by the following method. Specifically, a DNA encoding the homologous protein can be obtained, for example, by a method similar to a method in which a nucleotide sequence having 90% or more, preferably 91% or more, 92% or more, 93% or more, 94% or more, or 95% or more, more preferably 96% or more, 97% or more, or 98% or more, and further preferably 99% or more identity to the nucleotide sequence set forth in SEQ ID NO: 2 or 4 is searched for in various gene sequence database, and a probe DNA or a primer DNA designable based on a nucleotide sequence or an amino acid sequence obtained through the search, and a microorganism having the DNA are used to obtain the DNA encoding the protein consisting of the amino acid sequence set forth in SEQ ID NO: 3 or 5 above.

The identity between nucleotide sequences or amino acid sequences can be determined in the same manner as described above in item 1. It can be confirmed by a method similar to that described above in the item 1 that the mutant protein or the homologous protein encoded by the DNA of the present invention has NAPRT activity.

The DNA of the present invention obtained by any of the above-described methods is incorporated, directly or after cleaving with an appropriately restriction enzyme, into a vector by a conventional method, the thus obtained recombinant DNA is introduced into a host cell, and then, analysis is performed by a usually employed nucleotide sequence analysis method such as deoxy method [Proc. Nat. Aca. Sci., USA, 74, 5463 (1977)], or with a nucleotide sequence analyzer such as Applied Biosystems 3500 Genetic Analyzer or Applied Biosystems 3730 DNA analyzer (both manufactured by Thermo Fisher Scientific), and thus, the nucleotide sequence of the DNA can be determined.

Examples of the vector usable in determining the nucleotide sequence of the DNA of the present invention include pBluescript II KS (+), and pPCR-Script Amp SK (+) (both manufactured by Agilent Technologies Japan, Ltd.), pT7 Blue (manufactured by Merck Millipore), pCRII (manufactured by Thermo Fisher Scientific), pCR-TRAP (manufactured by Gene Hunter), and pDIRECT [Nucleic Acids Res., 18, 6069 (1990)].

The host cell may be any cell as long as it can be amplified with the vector introduced thereinto, and examples include *Escherichia coli* DH5α, *Escherichia coli* HST08Premium, *Escherichia coli* HST02, *Escherichia coli* HST04 dam-/dcm-, *Escherichia coli* JM109, *Escherichia coli* HB101, *Escherichia coli* CJ236, *Escherichia coli* BMH71-18 mutS, *Escherichia coli* MV1184, and *Escherichia coli* TH2 (all manufactured by Takara Bio Inc.), *Escherichia coli* XL1-Blue, and *Escherichia coli* XL2-Blue (both manufactured by Agilent Technologies Japan, Ltd.), *Escherichia coli* DH1, *Escherichia coli* MC1000, *Escherichia coli* W1485, *Escherichia coli* W3110, *Escherichia coli* MP347, and *Escherichia coli* NM522.

As a method for introducing the recombinant DNA, obtained by incorporation of the DNA of the present invention, into the host cell, any methods for introducing a DNA into a host cell can be employed, and examples include a method using a calcium ion [Proc. Natl. Acad. Sci., USA, 69, 2110 (1972)], Protoplast method (Japanese Unexamined Patent Publication No. S63-248394), and electroporation method [Nucleic Acids Res., 16, 6127 (1988)].

As a result of the determination of the nucleotide sequence, when the obtained DNA is a partial length DNA, a full length DNA can be obtained by Southern Hybridization in chromosomal DNA library using the partial length DNA as a probe, or the like.

Besides, based on the determined nucleotide sequence of the DNA, a target DNA can be prepared by chemical synthesis using NTSM series DNA Synthesizer manufactured by Nihon Techno Service Co., Ltd., or the like

### 3. Recombinant DNA of the present invention

A recombinant DNA of the present invention contains the DNA of the present invention. A recombinant DNA of the present invention is a DNA autonomous replicable in a host cell, and is a DNA in which the DNA of the present invention is incorporated into an expression vector containing a promoter in position where the DNA of the present invention described above in item 2 can be transcribed.

The recombinant DNA of the present invention is a DNA that can be incorporated into a chromosome in a host cell, and a DNA having the DNA of the present invention is also the recombinant DNA of the present invention.

When the recombinant DNA is a recombinant DNA that can be incorporated into a chromosome, a promoter may be or may not be contained therein.

When a prokaryote such as a bacterium is used as the host cell, the recombinant DNA of the present invention is preferably a recombinant DNA containing a promoter, a ribosome binding sequence, the DNA of the present invention described above in item 2, and a transcription termination sequence. A gene for controlling the promoter may be further contained therein.

Here, a distance between a Shine-Dalgarno sequence, that is, a ribosome binding sequence, and a start codon is preferably adjusted to an appropriate distance, such as 6 to 18 bases.

Besides, in the recombinant DNA of the present invention, a transcription termination sequence is not always necessary for expressing the DNA of the present invention, but a transcription termination sequence is preferably placed directly below a structural gene.

The expression vector is not especially limited as long as it is an appropriate nucleic acid molecule for introducing a target DNA into a host, and amplifying and expressing it therein, and not only a plasmid but also, for example, a vector or a cosmid using an artificial chromosome or transposon may be used.

When a microorganism belonging to the genus *Escherichia* is used as the host cell into which the recombinant DNA of the present invention is to be introduced, the expression vector can be, for example, pColdI, pSTV28, pSTV29, and pUC118 (all manufactured by Takara Bio Inc.), pET21a, pCDF-1b, and pRSF-1b (all manufactured by Merck Millipore), pMAL-c5x (manufactured by New England Biolabs, Inc.), pGEX-4T-1, and pTrc99A (both manufactured by GE Healthcare Bioscience), pTrcHis, and pSE280 (both manufactured by Thermo Fisher Scientific), pGEMEX-1 (manufactured by Promega Corp.), pQE-30, pQE-60, and pQE80L (all manufactured by Qiagen K.K.), pET-3, pBluescriptII SK(+), and pBluescriptII KS(-) (all manufactured by Agilent Technologies Japan, Ltd.), pKYP10 (Japanese Unexamined Patent Publication No. S58-110600), pKYP200 [Agric. Biol. Chem., 48, 669 (1984)], pLSA1[Agric. Biol. Chem., 53, 277 (1989)], pGEL1 [Proc. Natl. Acad. Sci., USA, 82, 4306 (1985)], pTrS30 [prepared from *Escherichia coli* JM109/pTrS30 (FERM BP-5407)], pTrS32 [prepared from *Escherichia coli* JM109/pTrS32 (FERM BP-5408)], pTK31 [APPLIED AND ENVIRONMENTAL MICROBIOLOGY, 2007, Vol. 73, No. 20, p. 6378-6385], pPE167 (Appl. Environ. Microbiol. 2007, 73: 6378-6385), pPAC31 (WO98/12343), pUC19 [Gene, 33, 103 (1985)], pPA1 (Japanese Unexamined Patent Publication No. S63-233798), and the like.

When any of the above-described expression vectors is used, the promoter may be any promoter as long as it functions in a cell of a microorganism belonging to the genus *Escherichia,* and, for example, a promoter derived from *Escherichia coli,* a phage or the like, such as a trp promoter, a gapA promoter, a lac promoter, a PL promoter, a PR promoter, or a PSE promoter, can be used. Besides, an artificially designed/modified promoter, such as a promoter containing two tandem trp promoters, a tac promoter, a trc promoter, a lac T5 promoter, a lac T7 promoter, or a letI promoter, can be used.

When a coryneform bacterium is used as the host cell into which the recombinant DNA of the present invention is to be introduced, the expression vector can be, for example, pCG1 (Japanese Unexamined Patent Publication No. S57-134500), pCG2 (Japanese Unexamined Patent Publication No. S58-35197), pCG4 (Japanese Unexamined Patent Publication No. S57-183799), pCG11 (Japanese Unexamined Patent Publication No. S57-134500), pCG116, pCE54, and pCB101 (all Japanese Unexamined Patent Publication No. S58-105999), pCE51, pCE52, and pCE53 [all Molecular and General Genetics, 196, 175 (1984)], and the like.

When any of the above-described expression vectors is used, the promoter may be any promoter as long as it functions in a cell of a coryneform bacterium, and for example, P54-6 promoter [Appl. Microbiol. Biotechnol., 53, p. 674-679 (2000)] can be used.

When a yeast strain is used as the host cell into which the recombinant DNA of the present invention is to be introduced, the expression vector can be, for example, YEp13 (ATCC37115), YEp24 (ATCC37051), YCp50 (ATCC37419), pHS19, pHS15, and the like.

When any of the above-described expression vectors is used, the promoter may be any promoter as long as it functions in a cell of a yeast strain, and examples include promoters such as a PHO5 promoter, a PGK promoter, a GAP promoter, an ADH promoter, a gall promoter, a gal10 promoter, a heat shock polypeptide promoter, a MFα1 promoter, and a CUP1 promoter.

The recombinant DNA of the present invention can be produced, for example, by treating a DNA fragment prepared by the method described above in item 2 with a restriction enzyme to insert the resultant into downstream of the promoter of any of the above-described appropriate expression vectors.

Here, when bases are substituted in the nucleotide sequence of the DNA of the present invention to obtain an optimal codon for expression of the host cell, the expression level of the protein encoded by the DNA can be also improved. Information on codon usage in the host cell is available through public database.

### 4. Transformant of the present invention

A transformant of the present invention is a transformant obtained by transforming a host cell with the recombinant DNA described above in item 3 containing the DNA of the present invention described above in item 2. The transformant of the present invention is preferably a transformant having increased productivity of nicotinamide mononucleotide (NMN) as compared with the host cell.

The host cell into which the recombinant DNA of the present invention is to be introduced may be any one of a prokaryote, a yeast, an animal cell, an insect cell, a plant cell, and the like, and may be preferably a prokaryote and a yeast strain, more preferably prokaryotes belonging to the genus *Escherichia,* the genus *Serratia,* the genus *Bacillus,* the genus *Brevibacterium,* the genus *Corynebacterium,* the genus *Microbacterium,* and the genus *Pseudomonas,* and yeast strains belonging to the genus *Saccharomyces,* the genus *Schizosaccharomyces,* the genus *Kluyveromyces,* the genus *Trichosporon,* the genus *Schwanniomyces,* the genus *Pichia,* and the genus *Candida,* and particularly preferably *E. coli* (*Escherichia coli*).

Specific examples of the preferable host cell include prokaryotes such as *Escherichia coli* BL21 codon plus, *Escherichia coli* XL1-Blue, and *Escherichia coli* XL2-Blue (all manufactured by Agilent Technologies Japan, Ltd.), *Escherichia coli* BL21(DE3) pLysS (manufactured by Merck Millipore), *Escherichia coli* DH5α, *Escherichia coli* HST08Premium, *Escherichia coli* HST02, *Escherichia coli* HST04 dam-/dcm-, *Escherichia coli* JM109, *Escherichia coli* HB101, *Escherichia coli* CJ236, *Escherichia coli* BMH71-18 mutS, *Escherichia coli* MV1184, and *Escherichia coli* TH2 (all manufactured by Takara Bio Inc.), *Escherichia coli W, Escherichia coli* JM101, *Escherichia coli* W3110, *Escherichia coli* MG1655, *Escherichia coli* DH1, *Escherichia coli* MC1000, *Escherichia coli* W1485, *Escherichia coli* MP347, *Escherichia coli* NM522, *Escherichia coli* ATCC9637, *Serratia ficaria, Serratia fonticola, Serratia liquefaciens, Serratia marcescens, Bacillus subtilis, Bacillus amyloliquefaciens, Brevibacterium immariophilum* ATCC14068, *Brevibacterium saccharolyticum* ATCC14066, *Corynebacterium ammoniagenes, Corynebacterium glutamicum* ATCC13032, *Corynebacterium glutamicum* ATCC14067, *Corynebacterium glutamicum* ATCC13869, *Corynebacterium acetoacidophilum* ATCC13870, *Microbacterium ammoniaphilum* ATCC15354, and *Pseudomonas* sp.D-0110, and yeast strains such as *Saccharomyces cerevisiae, Schizosaccharomyces pombe, Kluyveromyces lactis, Trichosporon pullulans, Schwanniomyces alluvius, Pichia pastoris,* and *Candida utilis.*

As the host cell, a bred strain artificially reduced in nicotinamide or β-nicotinamide mononucleotide degrading activity, a bred strain artificially imparted with or enhanced in productivity of nicotinamide to be used as a substrate of a target β-nicotinamide mononucleotide, or a bred strain having been subjected to both of these artificial treatments can be preferably used.

An example of a method for artificially reducing nicotinamide or β-nicotinamide mononucleotide degrading ability of a cell to be used as the host cell, particularly of a microorganism, includes a method in which at least one of enzymes having nicotinamide or β-nicotinamide mononucleotide degrading ability is reduced or blocked.

Examples of a method for artificially imparting with or enhancing in the ability to produce (productivity of) nicotinamide in a cell used as the host cell, particularly a microorganism, include a method (a) in which at least one of mechanisms for controlling a biosynthetic pathway for producing target nicotinamide is released or cancelled, a method (b) in which at least one of enzymes involved in the biosynthetic pathway for producing the target nicotinamide is enhanced in expression, a method (c) in which a copy number of at least one of enzyme genes involved in the biosynthetic pathway for producing the target nicotinamide is increased, and a method (d) in which at least one of metabolic pathways branching from the biosynthetic pathway for producing the target nicotinamide to metabolites other than the target substance is reduced or blocked, and these known methods can be singly used, or can be used in combination.

Specific examples of the above-described method for reducing nicotinamide or β-nicotinamide mononucleotide degrading activity, and imparting with or enhancing in the productivity of nicotinamide to be used as a substrate include known methods such as methods by various genetic engineering techniques (International Publication No. WO2018/211028).

Specifically, for example, as described in Non Patent Literature 5, for purpose of further improving the productivity of NMN using *E. coli* as the host cell, NiaP that is an intake system of nicotinamide, NMN excretory system PnuC, and PRPP synthesis enzyme PrsA may be enhanced.

Besides, as disclosed by William B. Black et al., (Microbial Cell Factories 19 (2020) 150), pncC that is NMN degradation system may be disrupted. NMN synthesis system NRK1 may be enhanced, or NAD repressor nadR may be disrupted, and the object of these treatments is mainly block of NMN degradation system.

Grose et al., (Journal of Bacteriology 187 (2005), 4521-4530) have reported aphA as NMN degradation system of salmonella, and pncA as degradation system of nicotinamide used as a substrate, and therefore the destruction of these enzymes also present in *E. coli* is expected to improve the productivity of NMN.

Besides, Chakwan Siew et al., (Journal of Biological Chemistry 286 (2011) 40365-40375) have reported on pncC that is NMN degradation system, Yang Liu et al., (Microbial Biotechnology 14 (2021) 2581-2591) have mentioned destruction of purR that is a regulator of purine nucleic acid, and destruction of AMP degrading enzyme amn for improving PRPP supply, and Patent Literature 3 discloses that various degradations are inhibited by disrupting ushA, deoD, rihA, rihB, and rihC in addition to the above strains. Therefore, it is presumed that the disruption of these enzymes leads to improvement of the NMN productivity.

Examples of a method for introducing the recombinant DNA described above in item 3 into a host cell as an autonomous replicable plasmid include the method using a calcium ion, the Protoplast method, and the electroporation method described above, and a spheroplast method [Proc. Natl. Acad. Sci., USA, 81, 4889 (1984)], and a lithium acetate method [J. Bacteriol., 153, 163 (1983)].

An example of a method for incorporating the recombinant DNA into a chromosome of a host cell includes a homologous recombination method. An example of the homologous recombination method includes a method using a homologous recombination plasmid producible to be linked to a plasmid DNA having a drug resistance gene that cannot be autonomously replicated in a host cell into which it is desired to be introduced. An example of a method utilizing homologous recombination, which is frequently used in *Escherichia coli,* includes a method for introducing a recombinant DNA utilizing a homologous recombination system of random phage [Proc. Natl. Acad. Sci. USA, 97, 6641-6645 (2000)].

Besides, *E. coli* in which a target region on a chromosomal DNA of a host cell is substituted with the recombinant DNA can be obtained by a selection method utilizing that *E. coli* attains sucrose sensitivity with *Bacillus subtilis* levansucrase incorporated into a chromosome together with a recombinant DNA, a selection method utilizing that *E. coli* attains streptomycin sensitivity through incorporation of wild type rpsL gene into *E. coli* having streptomycin-resistant mutant rpsL gene [Mol. Microbiol., 55, 137 (2005), Biosci. Biotechnol. Biochem., 71, 2905 (2007)], or the like.

It can be confirmed, by culturing the transformant in a medium to compare an amount of a target NMN produced, accumulated in the culture with that of a parent strain (host cell), that the transformant obtained by any of the above-described methods has the DNA of the present invention described above in item 2. Alternatively, it can be confirmed by preparing an extract containing the protein of the present invention from the culture, causing the extract and two different types of nicotinamide to be present in an aqueous medium, and comparing an amount of the NMN produced, accumulated in the aqueous medium with that of a parent strain.

Examples of such transformant of the present invention include transformants described below in examples.

### 5. Method for Producing Nicotinamide Mononucleotide of the present invention

An example of a method for producing nicotinamide mononucleotide (NMN) of the present invention includes a method for producing NMN by fermentation process in which a microorganism having the ability to produce the protein of the present invention described above in the item 1 is cultured in a medium to produce NMN in a culture. This production method may include, for example, after producing NMN in the culture, accumulating the NMN, and collecting the NMN from the culture.

As the microorganism used in the method for producing NMN by the fermentation process, the transformant of the present invention described above in the item 4 is preferably used, and a transformant having the ability to produce nicotinamide used as the substrate of NMN, and/or a transformant artificially reduced in nicotinamide or NMN degrading activity is preferred.

A method for culturing the transformant described above in item 4 can be performed in accordance with a usual method employed in culturing a microorganism.

As the medium for culturing the transformant, either of a natural medium and a synthetic medium may be used as long as it contains a carbon source, a nitrogen source, inorganic salts or the like that the transformant can assimilate, and the transformant can be efficiently cultured therein. The carbon source may be any source as long as the transformant can assimilate it, and sugars such as glucose, fructose, sucrose, syrup containing any of these, starch, and starch hydrolysate, organic acids such as acetic acid and propionic acid, and alcohols such as glycerol, ethanol, and propanol can be used.

As the nitrogen source, for example, ammonium salts of inorganic acids or organic acids such as ammonia, ammonium chloride, ammonium sulfate, ammonium acetate, and ammonium phosphate, other nitrogencontaining compounds, peptone, meat extract, yeast extract, corn steep liquor, casein hydrolysate, soybean cake, soybean cake hydrolysate, various fermented bacterial cells and digested materials thereof, and the like can be used.

As the inorganic salt, monopotassium phosphate, dipotassium phosphate, magnesium phosphate, magnesium sulfate, sodium chloride, ferrous sulfate, manganese sulfate, copper sulfate, calcium carbonate and the like can be used.

In the method for producing NMN by the fermentation process, when the transformant to be used does not have the ability to produce nicotinamide to be used as the substrate, the nicotinamide is added to the medium during the culturing.

When the transformant used in the method for producing NMN by fermentation process does not have ability to produce nicotinamide to be used as a substrate, nicotinamide may be supplied to the transformant of the present invention by co-culturing, with the transformant of the present invention, a microorganism having ability to produce nicotinamide during the culturing instead of adding nicotinamide to the medium.

The culturing is preferably performed usually by shaking culture or under aerobic conditions such as aerated and agitated culture. A culturing temperature is usually 15 to 40°C, and a culturing time is usually 5 hours to 7 days. The pH of the culture fluid during the culturing is usually kept at 3.0 to 9.0. The pH is adjusted using an inorganic or organic acid, an alkaline solution, urea, calcium carbonate, ammonia, or the like.

Besides, during the culturing, an antibiotic such as ampicillin or tetracycline may be added to the medium if necessary. In culturing a microorganism obtained by transformation with an expression vector using an inducible promoter as a promoter, an inducer may be added to the medium if necessary. For example, in culturing a microorganism obtained by transformation with an expression vector using a lac promoter, isopropyl-β-D-thiogalactopyranoside (IPTG) or the like may be added, and in culturing a microorganism obtained by transformation with an expression vector using a trp promoter, indoleacrylic acid or the like may be added to the medium.

NMN can be produced by producing NMN in the culture by performing the above culturing. Specifically, NMN can be produced, for example, by producing and accumulating NMN in the culture, and collecting the NMN from the culture.

The NMN thus obtained can be analyzed by a usual method using high performance liquid chromatography (HPLC) or the like. The collection of NMN from the culture or a treated product of the culture can be performed by a usual method using activated carbon or an ion exchange resin. When the NMN is accumulated in a bacterial cell, the bacterial cell is crushed ultrasonically or the like, for example, and the NMN can be collected, with activated carbon, an ion exchange resin or the like, from a supernatant obtained by removing the bacterial cell by centrifugation.

The method for producing NMN of the present invention may be a method for producing NMN using the protein of the present invention. NMN can be produced by reacting the protein of the present invention with nicotinamide, that is, the substrate of the protein.

Examples of the present invention will now be described, and it is noted that the present invention is not limited to these examples.

### Examples

### [Analysis Example] Analysis and Quantitative Determination of NMN

In each example, analysis and quantitative determination of NMN were performed with an analyzer, SPD-20AV (manufactured by Shimadzu Corporation).

### (Analysis Conditions)

Column: Shodex Asahipak MN2P-50 4E 4.6Φ, 250 mm, 5 µm (manufactured by Showa Denko K.K.)
Column temperature: 30°C
Mobile phase: 50 mM ammonium formate (pH 4.0):acetonitrile = 60:40 (v/v)
Flow rate: 0.6 ml/min
Detection wavelength: 260 nm

### [Example 1] Creation of Microorganisms Expressing Various NAMPTs

### (1) Obtaining of DNA encoding NAMPT

PCR was performed with each DNA shown in "Template" in Table 1 used as a template, and by using "Primer Set" shown in Table 1 to amplify a target DNA fragment.

**[Table 1]**

| Template | Primer Set (SEQ ID NO:) | Target DNA Fragment |
|---|---|---|
| DNA set forth in SEQ ID NO: 2 | 5'-cacaggaaacagaccatgtttaacctgcagcatagcac- 3' (SEQ ID NO: 13) | BhnadV (SEQ ID NO: 2) |
| | 5'-tactgccgccaggcattagccattcagttcactcagcatg- 3' (SEQ ID NO: 14) | |
| Chromosomal DNA of Chitinophaga rupis | 5'-cacaggaaacagaccatgcgtatcaatcccattctc-3' (SEQ ID NO: 15) | CrnadV (SEQ ID NO: 4) |
| | 5'-tactgccgccaggcattaaacaccggcgctcactg-3' (SEQ ID NO: 16) | |

A DNA set forth in SEQ ID NO: 2 is a DNA obtained by codon optimization, for expression in *E. coli,* of a nucleotide sequence (SEQ ID NO: 1) of a gene encoding *Bisgaardia hudsonensis*-derived nicotinamide phosphoribosyl transferase nadV (hereinafter referred to as "BhnadV"), and was prepared by artificial synthesis. The chromosomal DNA of *Chitinophaga rupis* was prepared by a usual method. CrnadV (SEQ ID NO: 4) is a gene encoding *Chitinophaga rupis*-derived nadV (SEQ ID NO: 5) (hereinafter referred to as "CrnadV"). The GenBank Accession number of a genomic DNA of *Bisgaardia hudsonensis* M327/99/2 is CP016605.1, and the GenBank Accession number of a genomic DNA of *Chitinophaga rupis* DSM 21039 is NZ_FOBB01000001.1.

PCR was performed with expression vector pTrc99A (E Amann, B Ochs, K J Abel 1988 Gene 30; 69(2): 301-315) used as a template, and with DNA fragments consisting of nucleotide sequences set forth in SEQ ID NOS: 17 and 18 as a primer set to obtain a vector fragment of about 4.0 kb.
Forward primer: 5'-tgcctggcggcagtagcg-3' (SEQ ID NO: 17)
Reverse primer: 5'-ggtctgtttcctgtgtgaaat-3' (SEQ ID NO: 18)

Each of the nucleotide sequences set forth in SEQ ID NOS: 13, 15, and 18, and SEQ ID NOS: 14, 16, and 17 contains a sequence complementary to the 3' end thereof.

The obtained amplified DNA fragment containing BhnadV or CrnadV was linked to the vector fragment with In-Fusion HD Cloning Kit (manufactured by Takara Bio Inc.) to create pBhnadV and pCrnadV, plasmids expressing respective NAMPTs.

### (2) Creation of Co-expression Plasmid

A NAMPT expression plasmid, in which a gene encoding the respective NAMPTs obtained as described above in the item (1), and a gene encoding *Escherichia* coli-derived ribose-phosphate diphosphokinase (PrsA) were placed below trc promoter was created as follows. The GenBank Accession number of a genomic DNA of *Escherichia coli* BL21 is CP053602.1.

PCR was performed with the expression vector pTrc99A used as a template, and with DNAs consisting of nucleotide sequences set forth in SEQ ID NOS: 19 and 18 as a primer set to obtain a trc promoter fragment of about 250 bp. PCR was performed with a chromosomal DNA of *Escherichia coli* BL21 prepared by a usual method used as a template, and with DNAs consisting of nucleotide sequences set forth in SEQ ID NOS: 20 and 21 used as a primer set to obtain a prsA fragment of about 950 bp.
Forward primer: 5'-gcgcgaattgatctggtttgacagcttatcatcg-3' (SEQ ID NO: 19)
Reverse primer: 5'-ggtctgtttcctgtgtgaaat-3' (SEQ ID NO: 18)
Forward primer: 5'-tttcacacaggaaacagaccatgaagctttttgctggtaacg-3' (SEQ ID NO: 20)
Reverse primer: 5'-tttcacacaggaaacagaccatgaagctttttgctggtaacg-3' (SEQ ID NO: 21)

PCR was performed with the trc promoter fragment and the prsA fragment obtained as described above used as a template, and with DNAs consisting of nucleotide sequences set forth in SEQ ID NOS: 19 and 21 used as a primer set to obtain a DNA fragment of about 1200 bp (hereinafter referred to as the Ptrc-prcA fragment).

PCR was performed with the pBhnadV and the pCrnadV created as described above in the item (1) used as templates, and with DNAs consisting of nucleotide sequences set forth in SEQ ID NOS: 22 and 23 used as a primer set to obtain vector fragments each of about 5.5 kb (hereinafter referred to as the pBhnadV fragment and the pCrnadV fragment).
Forward primer: 5'-gtttgacagcttatcatcgac-3' (SEQ ID NO: 22)
Reverse primer: 5'-cagatcaattcgcgctaactc-3' (SEQ ID NO: 23)

The Ptrc-prsA fragment and the pBhnadV fragment or the pCrnadV fragment obtained as described above were linked with In-Fusion HD Cloning Kit (manufactured by Takara Bio Inc.) to obtain pPrsA-BhnadV and pPrsA-CrnadV, that is, plasmids co-expressing the respective NAMPTs and PrsA.

### (3) Creation of E. coli having Co-expression Plasmid

*Escherichia coli* W3110 was transformed using the respective co-expression plasmids obtained as described above in the item (2) to create *E. coli* having the respective plasmids, which were respectively named W3110/pPrsA-BhnadV and W3110/pPrsA-CrnadV.

### [Comparative Example 1] Creation of Microorganism Expressing Known NAMPT

### (1) Obtaining of NAMPT Sequence

PCR was performed with DNAs shown in "Template" of Table 2 used as a template, and by using "Primer Set" shown in Table 2 to amplify target DNA fragments.

**[Table 2]**

| Template | Primer Set (SEQ ID NO:) | Target DNA Fragment |
|---|---|---|
| Chromosomal DNA of Shewanella oneidensis | 5'-cacaggaaacagaccatgtacttgaatcccgttactgc-3' (SEQ ID NO: 24) | SonadV (SEQ ID NO: 6) |
| | 5'-tactgccgccaggcatcagcgacgagacgctgccaatc-3' (SEQ ID NO: 25) | |
| DNA set forth in SEQ ID NO: 9 | 5'-cacaggaaacagaccatggacaatcttcttaactatagcag- 3' (SEQ ID NO: 26) | HdnadV (SEQ ID NO: 9) |
| | 5'-tactgccgccaggcattataaagtcgtacgggaaaccag-3' (SEQ ID NO: 27) | |

A chromosomal DNA of *Shewanella oneidensis* was prepared by a usual method. A DNA set forth in SEQ ID NO: 6 encodes *Shewanella oneidensis*-derived nicotinamide phosphoribosyl transferase nadV (hereinafter referred to as "SonadV") set forth in SEQ ID NO: 7. A DNA set forth in SEQ ID NO: 9 is a DNA obtained by codon optimization, for expression in *E. coli,* of a nucleotide sequence (SEQ ID NO: 8) of a gene encoding *Haemophilus ducreyi*-derived nicotinamide phosphoribosyl transferase nadV (hereinafter referred to as "HdnadV") set forth in SEQ ID NO: 10, and was prepared by artificial synthesis. Each of the nucleotide sequences set forth in SEQ ID NOS: 24, 26, and 18, and SEQ ID NOS: 25, 27, and 17 contains a sequence complementary to the 5' end thereof. The GenBank Accession number of a genomic DNA of *Shewanella oneidensis* MR-1 is CP053946.1, and the GenBank Accession number of a genomic DNA of *Haemophilus ducreyi* FDAARGOS 297 is CP022037.2.

The obtained amplified DNA fragments containing SonadV or HdnadV were linked to the vector fragment prepared as described above in the item (1) of Example 1 with In-Fusion HD Cloning Kit (manufactured by Takara Bio Inc.) to create pSonadV and pHdnadV, plasmids expressing respective NAMPTs.

### (2) Creation of Co-expression Plasmid

A gene encoding respective NAMPTs, and a gene encoding *Escherichia* coli-derived ribose-phosphate diphosphokinase (PrsA) were placed below trc promoter to create expression plasmids for expressing these genes as follows.

PCR was performed with pSonadV and pHdnadV created as described above in the item (1) used as a template, and with DNAs consisting of nucleotide sequences set forth in SEQ ID NOS: 22 and 23 used as a primer set to obtain vector fragments each of about 5.5 kb (hereinafter referred to as the pSonadV fragment and the pHdnadV fragment).

The Ptrc-prsA fragment obtained as described above in the item (2) of Example 1 and the pSonadV fragment or the pHdnadV fragment were linked with In-Fusion HD Cloning Kit (manufactured by Takara Bio Inc.) to create pPrsA-SonadV and pPrsA-HdnadV, plasmids co-expressing the respective NAMPTs and PrsA.

### (3) Creation of E. coli having Co-expression Plasmid

*Escherichia coli* W3110 was transformed using the co-expression plasmids obtained as described above to create *E. coli* having the respective plasmids, which were respectively named W3110/pPrsA-SonadV and W3110/pPrsA-HdnadV.

### [Example 2] Production of NMN

The W3110/pPrsA-BhnadV and the W3110/pPrsA-CrnadV created in Example 1 were evaluated for the productivity of NMN. As a positive control, W3110/pPrsA-SonadV and W3110/pPrsA-HdnadV created in Comparative Example 1 were used.

Each of these strains was inoculated into a large test tube holding 5 mL of an LB medium [10 g/L of bacto tryptone (manufactured by Difco Laboratories Inc.), 5 g/L of yeast extract (manufactured by Difco Laboratories Inc.), 10 g/L of sodium chloride] containing 100 mg/L of ampicillin to be shaking cultured at 30°C for 20 hours. 1% of the resultant culture fluid was inoculated into a large test tube holding 5 mL of an LB medium containing 100 mg/L of ampicillin to be shaking cultured at 30°C for 16 hours. Two hours after starting the culturing, IPTG was added thereto to a final concentration of 0.5 mM.

After completing the culturing, 5 mL of each of the resultant culture fluids was centrifuged, and a supernatant was removed to obtain a wet bacterial cell. The entire amount of the wet bacterial cell was suspended in a large test tube holding 5 mL of a reaction medium (3 g/L of potassium dihydrogen phosphate, 6.8 g/L of sodium dihydrogen phosphate, 0.5 g/L of sodium chloride, 1 g/L of ammonium chloride, 20 g/L of glucose, 0.25 g/L of magnesium sulfate heptahydrate, 14.7 mg/L of calcium chloride, 2.6 g/L of NAM) to be shaken at 30°C for 2 hours for performing the reaction. After the reaction, the resultant reaction solution was centrifuged, and a supernatant thus obtained was used in analysis ofNMN. The test was independently conducted 3 times. The results are shown in Table 3.

**[Table 3]**

| | SonadV | HdnadV | BhnadV | CrnadV |
|---|---|---|---|---|
| NMN(mg/L ) | 0.33±0.03 2 | 0.59±0.076 * | 11.96±0.121* * | 1.58±0.096* * |

| | | | | |
|---|---|---|---|---|
| Mean±SD; *: p<0.05 **: p<0.01 | | | | |

It was found, based on these results, that the BhnadV and the CrnadV have higher NMN production activity than the known NAMPTs, SonadV and HdnadV. In particular, the BhnadV has NMN productivity 10 times or more as high as the known NAMPTs, and it was revealed that NMN can be efficiently produced by using this enzyme.

## Claims

1. A protein having nicotinamide phosphoribosyl transferase activity, described in any one of the following [1] to [3]:
[1] a protein consisting of an amino acid sequence set forth in SEQ ID NO: 3 or 5;
[2] a mutant protein consisting of an amino acid sequence obtained by deleting, substituting, inserting, or adding 1 to 20 amino acids in the amino acid sequence set forth in SEQ ID NO: 3 or 5; and
[3] a homologous protein consisting of an amino acid sequence having 90% or more identity to the amino acid sequence set forth in SEQ ID NO: 3 or 5.

2. A DNA encoding the protein according to claim 1.

3. The DNA according to claim 2 that is a DNA described in any one of the following [4] to [6]:
[4] a DNA consisting of a nucleotide sequence set forth in SEQ ID NO: 2 or 4;
[5] a DNA that hybridizes, under stringent conditions, a DNA consisting of a nucleotide sequence complementary to the nucleotide sequence set forth in SEQ ID NO: 2 or 4; and
[6] a DNA consisting of a nucleotide sequence having 90% or more identity to the nucleotide sequence set forth in SEQ ID NO: 2 or 4.

4. A recombinant DNA comprising the DNA according to claim 2 or 3.

5. A transformant obtained by transforming a host cell with the recombinant DNA according to claim 4.

6. The transformant according to claim 5 that is a transformant having increased productivity of nicotinamide mononucleotide (NMN) as compared with the host cell.

7. The transformant according to claim 5, wherein the host cell is *E. coli.*

8. A method for producing NMN, comprising producing nicotinamide mononucleotide (NMN) by using the transformant according to claim 5.

9. A method for producing NMN, comprising producing nicotinamide mononucleotide (NMN) by using the protein according to claim 1.
